(19) Europäisches Patentamt / European Patent Office / Office européen des brevets

(11) **EP 1 576 922 B1**

(12) **EUROPEAN PATENT SPECIFICATION**

(45) Date of publication and mention
of the grant of the patent:
**22.08.2007 Bulletin 2007/34**

(51) Int Cl.:
**A61B 5/053** (2006.01)

(21) Application number: **05003677.1**

(22) Date of filing: **21.02.2005**

(54) **Skin condition estimating apparatus**

Vorrichtung zur Abschätzung des Hautzustandes

Dispositif pour estimer l'état de la peau

(84) Designated Contracting States:
**DE FR GB**

(30) Priority: **15.03.2004 JP 2004072285**

(43) Date of publication of application:
**21.09.2005 Bulletin 2005/38**

(73) Proprietor: **TANITA CORPORATION**
**Tokyo (JP)**

(72) Inventor: **Nakada, Masato,**
**Tanita Corporation**
**Itabashi-ku**
**Tokyo (JP)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**EP-A- 1 080 686**

• **SALTER D C: "Examination of stratum corneum hydration state by electrical methods" CURRENT PROBLEMS IN DERMATOLOGY (BASEL); SKIN BIOENGINEERING: TECHNIQUES AND APPLICATIONS IN DERMATOLOGY AND COSMETOLOGY S. KARGER AG, P.O. BOX, ALLSCHWILERSTRASSE 10, CH-4009 BASEL, SWITZERLAND; S. KARGER AG, NEW YORK, NEW YORK, USA SERIES : CURRENT, 1998, pages 38-47, XP008048739 ISSN: 3-8055-6519-4**
• **VILHUNEN T ET AL: "Simultaneous reconstruction of electrode contact impedances and internal electrical properties. I. Theory" MEASUREMENT SCIENCE & TECHNOLOGY IOP PUBLISHING UK, vol. 13, no. 12, December 2002 (2002-12), pages 1848-1854, XP002332619 ISSN: 0957-0233**
• **YAMAMOTO Y ET AL: "MEASUREMENT PRINCIPLE FOR EVALUATING THE PERFORMANCE OF DRUGS AND COSMETICS BY SKIN IMPEDANCE" MED BIOL ENG COMPUT NOV 1978, vol. 16, no. 6, November 1978 (1978-11), pages 623-632, XP002332620**

**Description**

BACKGROUND OF THE INVENTION

(i) Field of the Invention

The present invention relates to a skin condition estimating apparatus which estimates a skin condition in accordance with an impedance method.

(ii) Description of the Related Art

In recent years, as apparatuses which estimate a skin condition, there are disclosed apparatuses which measure the content of water in the skin with electrodes incorporated in a grip applied to the skin (refer to Patent Publications 1 and 2). These apparatuses determine the content of water in the skin by determining a change in the capacitance or resistance of the skin-forming horny layer which is a dielectric or resistor and allow a user to know a skin condition easily.

> Patent Publication 1
> Japanese Patent Laid-Open Publication No. 2003-169784
> Patent Publication 2
> Japanese Patent Laid-Open Publication No. 2003-169785

Meanwhile, in the current society which demands information of higher quality, it has been increasingly demanded to know a skin condition by a more sensory characteristic than the water content, e.g., moisture, resilience, texture and oiliness. Such more sensory characteristics as moisture, resilience, texture and oiliness are based not only on the horny layer but also on the overall cellular structure of the skin layer associated with formation of the skin. Therefore, the more sensory characteristics such as moisture, resilience, texture and oiliness cannot be determined by use of apparatuses such as those disclosed in the above Patent Publications 1 and 2 which make a measurement with the horny layer as a dielectric or resistor.

Document XP008048739 discloses the subject-matter according to the preamble of claim 1.

In view of the above problem of the prior art, an object of the present invention is to provide a skin condition estimating apparatus which allows a user to know a skin condition by more sensory characteristics.

SUMMARY OF THE INVENTION

A skin condition estimating apparatus of the present invention comprises:

> impedance measuring means, and
> skin condition estimating means,
> wherein
> the impedance measuring means has electrodes to make contact with a body and passes alternating currents of multiple frequencies through the body when the body is in contact with the electrodes to measure a contact impedance and an internal impedance which occur during the energization, and
> the skin condition estimating means estimates a skin condition based on at least one of the contact impedance measured by the impedance measuring means and the internal impedance measured by the impedance measuring means.

Further, the apparatus further comprises biological equivalent model associated parameter estimating means, wherein
the biological equivalent model associated parameter estimating means estimates parameters associated with an equivalent model constituting a living tissue based on the frequencies of the currents passed by the impedance measuring means and the measured internal impedance, and
the skin condition estimating means estimates a skin condition based on at least one of the contact impedance measured by the impedance measuring means, the internal impedance measured by the impedance measuring means and the parameters estimated by the biological equivalent model associated parameter estimating means.

Further, the parameters associated with the equivalent model constituting the living tissue are an extracellular fluid resistance, an intracellular fluid resistance, distributed membrane capacitance, a parallel combined resistance of the extracellular fluid resistance and the intracellular fluid resistance, and an angle formed by the real part axis and a straight line which connects the real part axis with an internal impedance measured when a vector locus corresponding to the internal impedances measured by the impedance measuring means using the alternating currents of multiple frequencies is drawn on the real part axis and the imaginary part axis based on the arc law representing a vector locus of impedances dependent on frequencies.

Further, the biological equivalent model associated parameter estimating means estimates the extracellular fluid resistance, the intracellular fluid resistance and the distributed membrane capacitance by use of the following equation:

$$Z_B \; (= \cdot R_B + j \times X_B) \; = \; \text{Re}(Ri + 1/2 \times \pi \times f \times j \times Cm)$$
$$/Re + (Ri + 1/2 \times \pi \times f \times j \times Cm)$$

wherein Re represents an extracellular fluid resistance, Ri represents an intracellular fluid resistance, Cm represents distributed membrane capacitance, f represents a frequency, j represents an imaginary number, $\pi$ represents a pi, and $Z_B$ represents an internal impedance ($R_B$ represents a resistance component and $X_B$ represents a reactance component).

Further, the alternating currents of multiple frequencies are an alternating current of 50 kHz and an alternating current of 6.25 kHz.

Further, the skin condition estimating means estimates at least one characteristic selected from the group consisting of moisture (or dryness), resilience (or swelling), texture and oiliness (or gloss) as a skin condition.

Further, the skin condition estimating means estimates moisture (or dryness), resilience (or swelling), texture and oiliness (or gloss) by use of the following equations:

$$Fw = a_{w1} \times R_{C50}^{-1} + a_{w2} \times Re^{-1} + a_{w3} \times Ri/Re + a_{w4}$$

$$Ft = a_{t1} \times Re^{-1} + a_{t2} \times R_\infty^{-1} + a_{t3} \times Ri^{-1} + a_{t4} \times \phi + a_{t5}$$

$$Fs = a_{s1} \times \phi + a_{s2} \times X_{B50}/R_{B50} + a_{s3}$$

$$Ff = a_{f1} \times R_{B6.25}/X_{B6.25} + a_{f2} \times R_{C50}/X_{C50} + a_{f3}$$

wherein Fw represents moisture (or dryness), Ft represents resilience (or swelling), Fs represents texture, Ff represents oiliness (or gloss), $R_{C50}$ represents the resistance component of a contact impedance with an alternating current of 50 kHz, $X_{C50}$ represents the reactance component of the contact impedance with the alternating current of 50 kHz, $R_{B50}$ represents the resistance component of an internal impedance with an alternating current of 50 kHz, $X_{B50}$ represents the reactance component of the internal impedance with the alternating current of 50 kHz, $R_{B6.25}$ represents the resistance component of an internal impedance with an alternating current of 6.25 kHz, $X_{B6.25}$ represents the reactance component of the internal impedance with the alternating current of 6.25 kHz, Re represents an extracellular fluid resistance, Ri represents an intracellular fluid resistance, $R_\infty$ represents a parallel combined resistance of the extracellular fluid resistance and the intracellular fluid resistance, $\phi$ represents an angle formed by the real part axis and a straight line which connects the real part axis with an internal impedance measured with an alternating current of 50 kHz when an arc corresponding to the internal impedances measured by the impedance measuring means using the alternating currents of multiple frequencies is drawn on the real part axis and the imaginary part axis based on the arc law representing the relationship of impedances dependent on frequencies, and $a_{w1}$ to $a_{w4}$, $a_{t1}$ to $a_{t5}$, $a_{s1}$ to $a_{s3}$ and $a_{f1}$ to $a_{f3}$ represent coefficients (constants).

The skin condition estimating apparatus according to the present invention passes alternating currents of multiple frequencies through the skin layer so as to measure a contact impedance and an internal impedance based on the overall cellular structure of the skin layer by the impedance measuring means and estimates a skin condition based on at least one of these impedances by the skin condition estimating means. Accordingly, the apparatus allows a user to know a skin condition by more sensory characteristics.

Further, the apparatus further estimates parameters associated with a living tissue forming the base of a skin condition by the biological equivalent model associated parameter estimating means and estimates a skin condition based on these parameters in addition to the above impedances by the skin condition estimating means. Therefore, the apparatus allows a user to know a skin condition by more sensory characteristics.

BRIEF DESCRIPTION OF THE DRAWINGS

Fig. 1 is a block diagram illustrating the functional constitution of a skin condition estimating apparatus.
Fig. 2 is a bioelectrical equivalent model.
Fig. 3 is a diagram illustrating a vector locus of impedances dependent on frequencies.
Fig. 4 is a diagram illustrating the appearance of the skin condition estimating apparatus.
Fig. 5 is a block diagram illustrating the structural constitution of the skin condition estimating apparatus.
Fig. 6 is a circuit model in measuring a body.
Fig. 7 is a main flowchart illustrating the flow of operations of the skin condition estimating apparatus in the constant acquiring mode.
Fig. 8 is a main flowchart illustrating the flow of operations of the skin condition estimating apparatus in the measuring mode.
Fig. 9 is a subroutine flowchart illustrating the flow of operations of the skin condition estimating apparatus in the constant acquiring mode.
Fig. 10 is a subroutine flowchart illustrating the flow of operations of the skin condition estimating apparatus in the measuring mode.

DETAILED DESCRIPTION OF THE PREFERRED EMBODIMENTS

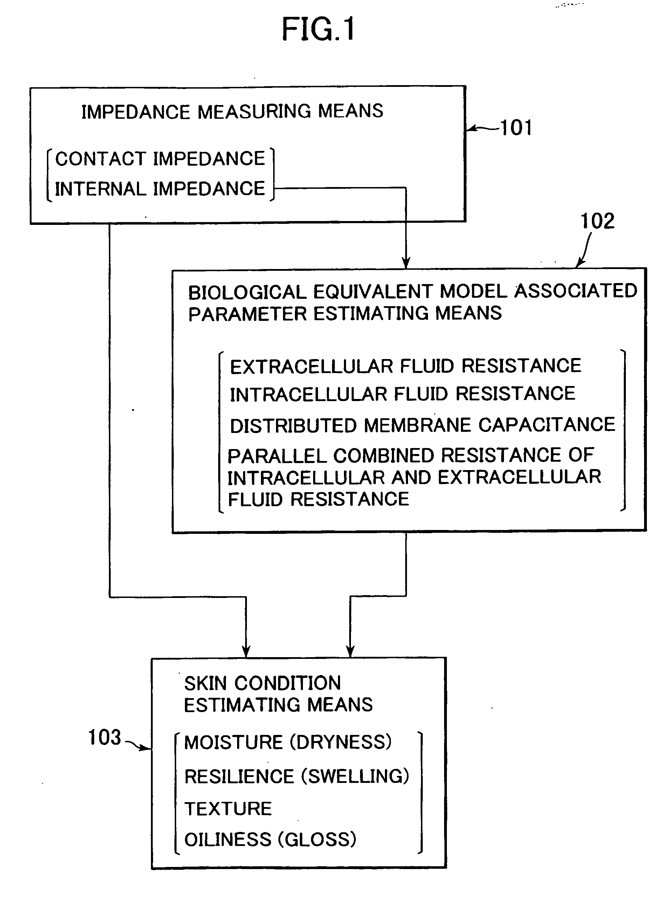

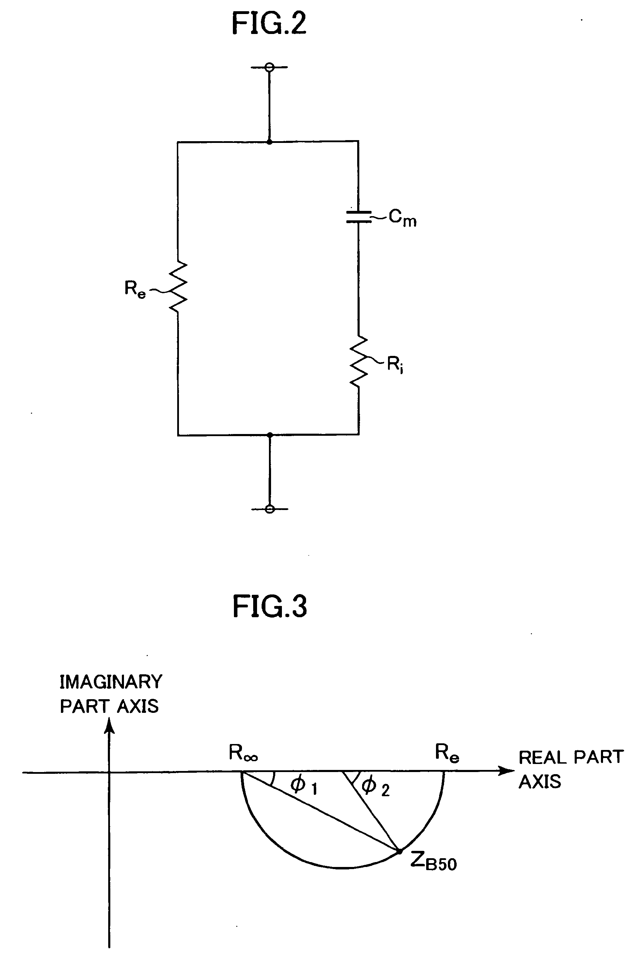

A skin condition estimating apparatus according to the present invention will be described in detail with reference to a functional constitution block diagram shown in Fig. 1, a bioelectrical equivalent model diagram shown in Fig. 2, and a diagram shown in Fig. 3 which illustrates a vector locus of impedances dependent on frequencies. As shown in Fig. 1, the skin condition estimating apparatus according to the present invention comprises impedance measuring means, biological equivalent model associated parameter estimating means, and skin condition estimating means.

The impedance measuring means has electrodes to make contact with a body and passes alternating currents of multiple frequencies through the body when the body is in contact with the electrodes to measure a contact impedance and an internal impedance which occur during the energization. More specifically, the impedance measuring means comprises a measuring section, a computing equation storing section, a constant computing section, a constant storing section, a contact impedance computing section and an internal impedance computing section and measures a contact impedance value and an internal impedance value.

The measuring section has a plurality of electrodes and an internal standard impedance and measures the following voltage values, i.e., (i) a first external standard voltage value generated between both ends of a first external standard impedance and a first internal standard voltage value generated between both ends of the internal standard impedance when the first external standard impedance is in contact with the electrodes, (ii) a second external standard voltage value generated between both ends of a second external standard impedance and a second internal standard voltage value generated between both ends of the internal standard impedance when the second external standard impedance is in contact with the electrodes, (iii) a third external standard voltage value generated between both ends of the first external standard impedance and a third internal standard voltage value generated between both ends of the internal standard impedance when the first external standard impedance and the third external standard impedance are in contact with the electrodes and (iv) a body voltage value generated between body parts in contact with the electrodes and a fourth internal standard voltage value generated between both ends of the internal standard impedance when a body is in contact with the electrodes. An internal impedance component of a body is assumed for the first external standard impedance and the second external standard impedance, and a contact impedance component is assumed for the third external standard impedance.

The computing equation storing section stores the following computing equations, i.e., (i) a first external standard impedance computing equation for computing a first external standard impedance value based on variation constants based on impedance variation factors, a first external standard voltage value and a first internal standard voltage value, (ii) a second external standard impedance computing equation for computing a second external standard impedance value based on the variation constants based on impedance variation factors, a second external standard voltage value and a second internal standard voltage value, (iii) an inclination computing equation for computing an inclination constant of an internal external standard impedance relationship based on the first external standard impedance value, the second external standard impedance value, a third external standard impedance value, a third internal standard voltage value and a second internal standard voltage value, (iv) a total impedance computing equation for computing a total impedance in measuring a body based on the inclination constant of the internal external standard impedance relationship, a fourth internal standard voltage value and a section constant (second internal standard voltage value) of the internal external standard impedance relationship, (v) a first provisional internal impedance computing equation for computing a provisional internal impedance in measuring the body based on the variation constants based on impedance variation factors, a body voltage value and the fourth internal standard voltage value, (vi) a body contact impedance computing equation for computing a contact impedance value in measuring the body based on the total impedance value and the provisional internal impedance value in measuring the body, (vii) a second provisional internal impedance computing equation for computing a provisional internal impedance value in measuring the first external standard impedance and the third external standard impedance based on the variation constants based on impedance variation factors, the third internal standard voltage value and a third external standard voltage value, (viii) an external standard contact impedance computing equation for computing a contact impedance value in measuring the external standard impedance based on the provisional internal impedance value in measuring the first external standard impedance and the third external standard impedance, the first external standard impedance value and the third external standard impedance value, (ix) an external standard impedance computing equation for computing a first external standard impedance value based on the provisional internal impedance value in measuring the first external standard impedance and the third external standard impedance, the contact impedance value in measuring the external standard impedance and a correction constant, and (x) an internal impedance computing equation for computing an internal impedance value in measuring the body based on the contact impedance value in measuring the body, the provisional internal impedance value in measuring the body and the correction constant. The first external standard impedance value, the second external standard impedance value, the third external standard impedance value and the internal standard impedance in the first external standard impedance computing equation, the second external standard impedance computing equation, the inclination computing equation, the external standard contact impedance computing equation and the external standard impedance computing equation are default values determined based on values expected in measuring a body.

The constant computing section computes the following constants. That is, the constant computing section computes (i) the variation constants based on impedance variation factors by substituting the first external standard voltage value and the first internal standard voltage value which have been measured by the measuring section into the first external standard impedance computing equation stored in the computing equation storing section and substituting the second external standard voltage value and the second internal standard voltage value which have been measured by the measuring section into the second external standard impedance computing equation stored in the computing equation storing section, (ii) the inclination constant of the internal external standard impedance relationship by substituting the third internal standard voltage value and the second internal standard voltage value which have been measured by the measuring section into the inclination computing equation stored in the computing equation storing section, and (iii) the correction constant by substituting the provisional internal impedance value in measuring the first external standard impedance and the third external standard impedance and the contact impedance value in measuring the external standard impedance which have been computed by the contact impedance computing section into the external standard impedance computing equation stored in the computing equation storing section.

The constant storing section stores the following constants, i.e., (i) the variation constants based on impedance variation factors which have been computed by the constant computing section, (ii) the inclination constant of the internal external standard impedance relationship which has been computed by the constant computing section, (iii) the second internal standard voltage value (section constant of the internal external standard impedance relationship) measured by the measuring section, and (iv) the correction constant computed by the constant computing section.

The contact impedance computing section computes the following impedance values, i.e., (i) the total impedance value in measuring the body by substituting the inclination constant of the internal external standard impedance relationship which has been stored in the constant storing section and the fourth internal standard voltage value and the second internal standard voltage value which have been measured by the measuring section into the total impedance computing equation stored in the computing equation storing section, (ii) the provisional internal impedance value in measuring the body by substituting the variation constants based on impedance variation factors which have been stored in the constant storing section and the body voltage value and the fourth internal standard voltage value which have been measured by the measuring section into the first provisional internal impedance computing equation stored in the computing equation storing section, (iii) the contact impedance value in measuring the body by substituting the above computed total impedance value in measuring the body and provisional internal impedance value in measuring the body into the body contact impedance computing equation stored in the computing equation storing section, (iv) the provisional internal impedance value in measuring the first external standard impedance and the third external standard impedance by substituting the third external standard voltage value and the third internal standard voltage value which have been measured by the measuring section and the variation constants based on impedance variation factors which have been stored in the constant storing section into the second provisional internal impedance computing equation stored in the computing equation storing section, and (v) the contact impedance (resistance component and reactance component) value in measuring the external standard impedance by substituting the provisional internal impedance value in measuring the first external standard impedance and the third external standard impedance into the external standard contact impedance computing equation stored in the computing equation storing section.

The internal impedance computing section computes the internal impedance (resistance component and reactance component) value in measuring the body by substituting the contact impedance value in measuring the body and the provisional internal impedance value in measuring the body which have been computed by the body contact impedance computing section and the correction constant stored in the constant storing section into the internal impedance computing equation stored in the computing equation storing section.

The biological equivalent model associated parameter estimating means estimates parameters associated with an equivalent model constituting a living tissue based on the frequencies of the currents passed by the impedance measuring means and the measured internal impedance (resistance component and reactance component). More specifically, the biological equivalent model associated parameter estimating means comprises the computing equation storing section (which is shared by the impedance measuring means) and an equivalent model associated parameter computing section. The equivalent model associated parameter computing section computes the values of an extracellular fluid resistance Re, an intracellular fluid resistance Ri and distributed membrane capacitance Cm as shown in Fig. 2 as parameters associated with an equivalent model constituting a living tissue by substituting the internal impedance value computed in the internal impedance computing section, the frequency of the current passed by the impedance measuring means and the like into a biological equivalent model associated parameter computing equation stored in the computing equation storing section and computes the value of an angle $\phi$ ($\phi_1$ or $\phi_2$) formed by the real part axis and a straight line which connects the real part axis with the internal impedance $Z_{B50}$ measured when a vector locus corresponding to internal impedances measured by the impedance measuring means using alternating currents of multiple frequencies is drawn on the real part axis and the imaginary part axis based on the arc law representing a vector locus of impedances dependent on frequencies and the value of a parallel combined resistance $R_\infty$ ($= Re//Ri$) of the extracellular fluid resistance and the intracellular fluid resistance as shown in Fig. 3 as parameters associated with an equivalent model constituting a living tissue by use of the above computed values, the internal impedance value computed in the internal impedance computing section and the frequency of the current passed by the impedance measuring means.

The skin condition estimating means estimates a skin condition based on at least one of the contact impedance measured by the impedance measuring means, the internal impedance measured by the impedance measuring means and the parameters associated with an equivalent model constituting a living tissue which have been estimated by the biological equivalent model associated parameter estimating means. More specifically, the skin condition estimating means comprises the computing equation storing section (which is shared by the impedance measuring means) and a skin condition computing section. The skin condition computing section computes the values of moisture (or dryness), resilience (or swelling), texture, oiliness (or gloss) and the like as characteristics representing a skin condition by substituting the contact impedance computed in the contact impedance computing section, the internal impedance value measured by the impedance measuring means, the internal impedance value computed in the internal impedance computing section and the values of the extracellular fluid resistance Re, intracellular fluid resistance Ri, distributed membrane capacitance Cm, angle $\phi$ and parallel combined resistance $R_\infty$ which have been computed in the equivalent model associated parameter computing section into skin condition computing equations stored in the computing equation storing section.

In the above embodiment, it is also possible that the biological equivalent model associated parameter estimating means is omitted and the characteristics representing a skin condition are estimated by use of only the internal impedance (resistance component and reactance component) value measured by the impedance measuring means in the skin condition estimating means.

Hereinafter, a specific example of the skin condition estimating apparatus according to the present invention will be described.

Examples

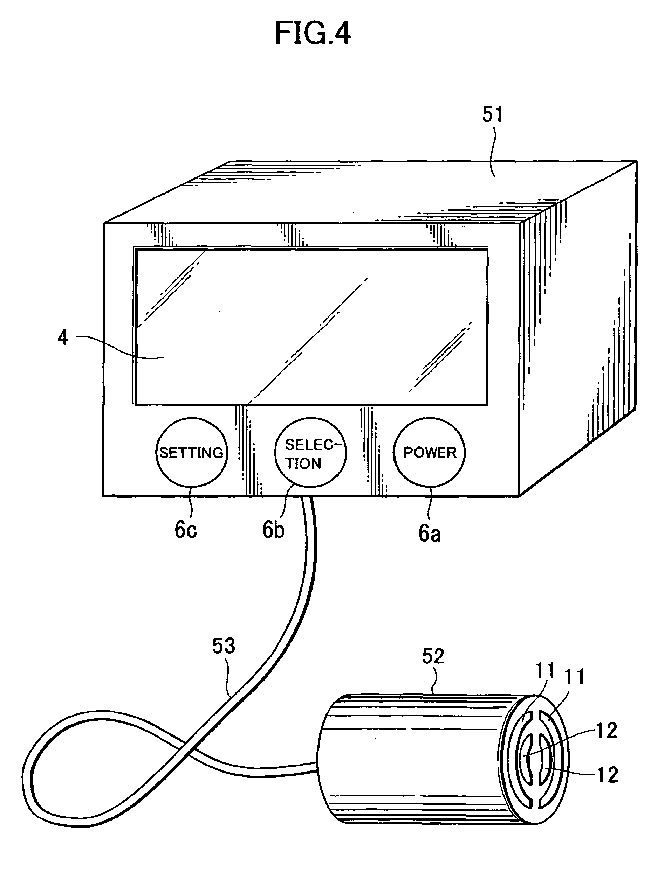

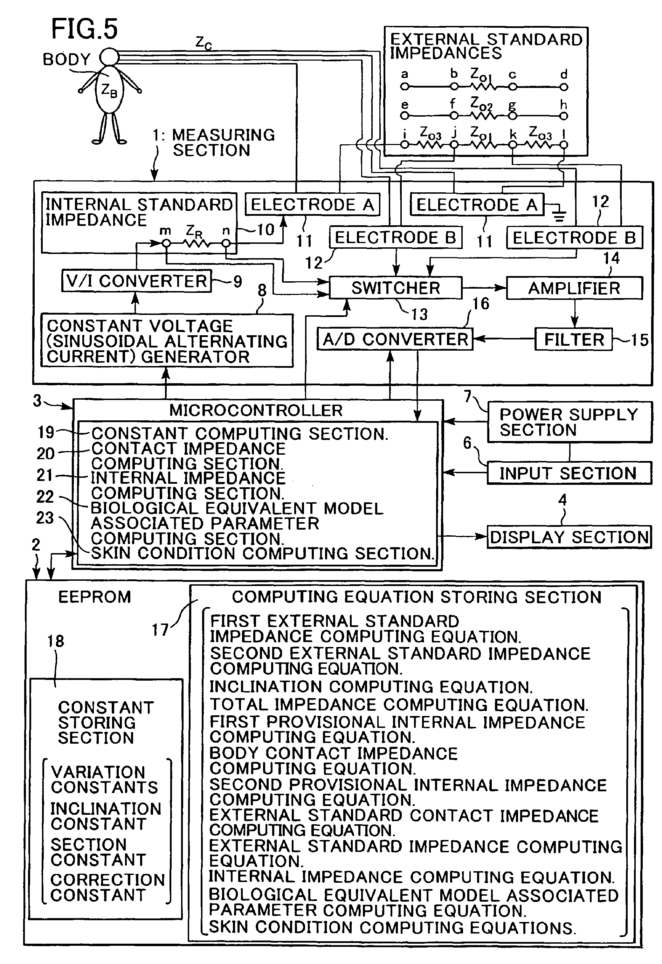

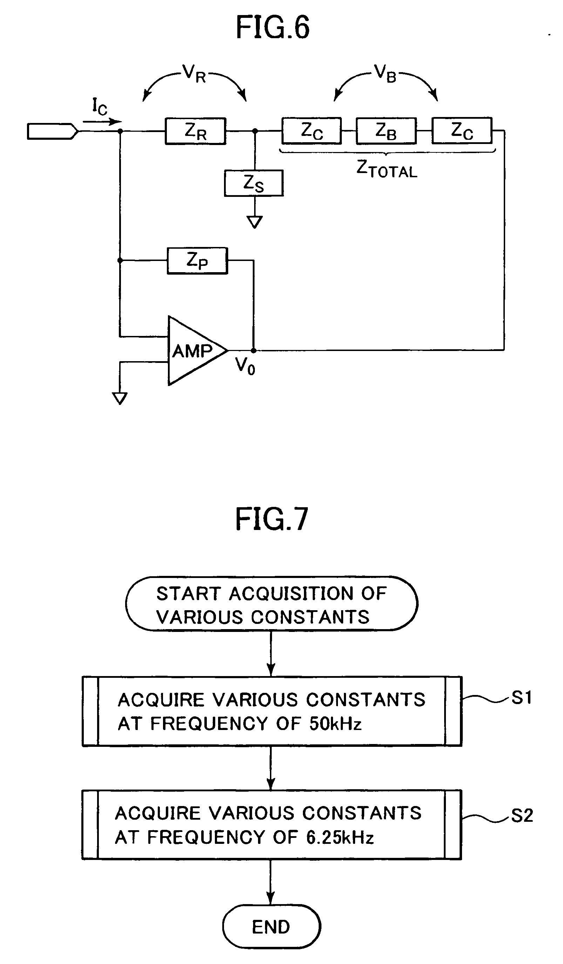

First, a specific constitution of the skin condition estimating apparatus according to the present invention will be described by use of an external view shown in Fig. 4, a structural block diagram shown in Fig. 5 and a circuit model diagram shown in Fig. 6.

The skin condition estimating apparatus comprises a chassis 51, a grip 52, and a cord 53 which connects the grip 52 to the chassis 51. The chassis 51 comprises a measuring section 1 excluding electrodes A 11 and B 12, an EEPROM 2, a microcontroller 3, a display section 4, an input section 6 (comprising a power key 6a, a selection key 6b and a setting key 6c), and a power supply section 7. The grip 52 comprises the electrodes A 11 and the electrodes B 12.

Of these sections constituting the skin condition estimating apparatus, the measuring section 1 measures a voltage value based on an impedance for a body, an external standard impedance (resistance) or an internal standard impedance (resistance). The measuring section 1 comprises a constant voltage (sinusoidal alternating current) generator 8, a V/I converter 9, an internal standard impedance 10, the electrodes A 11, the electrodes B 12, a switcher 13, an amplifier 14, a filter 15, and an A/D converter 16. In the present example, three 800-Ω resistances and one 200-Ω resistance are used for the external standard impedance, and one 800-Ω resistance is used for the internal standard impedance 10. These resistance values are determined based on values expected at the time of measurement of body (skin layer) .

Of these sections constituting the measuring section 1, the constant voltage (sinusoidal alternating current) generator 8 generates a high-frequency constant voltage. The V/I converter 9 converts a constant voltage received from the constant voltage (sinusoidal alternating current) generator 8 into a constant current.

The internal standard impedance 10 is referred to as a standard for correcting a change in constant current which is caused by a change in the temperature of an environment or the like, stray capacitance and a contact impedance which occurs when a body makes contact with the electrodes.

The electrodes A 11 are terminals for passing a constant current which is output from the V/I converter 9 and passed through the internal standard impedance 10 through a body or the external standard impedance. The electrodes B 12 are terminals for detecting a voltage generated in the body or external standard impedance. The electrodes A 11 and B 12 are disposed on an end face of the grip 52 which is not connected the cord 53.

The switcher 13 switches between detection of voltage value generated between both ends of the internal standard impedance when a constant current passes through the internal standard impedance 10 and detection of voltage value generated between the two electrodes B when a constant current passes through a body between the two electrodes B or the external standard impedance. More specifically, the switcher 13 switches (i) between detection of first external standard voltage value generated between both ends of the first external standard impedance and detection of first internal standard voltage value generated between both ends of the internal standard impedance when the first external standard impedance is in contact with the electrodes, (ii) between detection of second external standard voltage value generated between both ends of the second external standard impedance and detection of second internal standard voltage value generated between both ends of the internal standard impedance when the second external standard impedance is in contact with the electrodes, (iii) between detection of third external standard voltage value generated between both ends of the first external standard impedance and detection of third internal standard voltage value generated between both ends of the internal standard impedance when the first external standard impedance and the third external standard impedance are in contact with the electrodes and (iv) between detection of body voltage value generated between body parts in contact with the electrodes and detection of fourth internal standard voltage value generated between both ends of the internal standard impedance when a body is in contact with the electrodes.

After the switcher 13, the amplifier 14 amplifies the voltage value based on the impedance of the internal standard impedance 10 or the voltage value based on the impedance of the body or external standard impedance. The filter 15 removes a noise component from the voltage amplified by the amplifier 14. The A/D converter 16 digitizes the voltage (analog) denoised by the filter 15.

Of these sections constituting the skin condition estimating apparatus, the EEPROM 2 comprises a computing equation storing section 17 and a constant storing section 18 and stores various computing equations and various constants in advance. More specifically, the EEPROM 2 stores:

(i) a first external standard impedance computing equation:

$$Z_{O1} = 800 = c \times V_{O1}/V_{R1} + os$$

wherein c and os represent variation constants based on impedance variation factors, $V_{O1}$ represents a first external standard voltage value, $V_{R1}$ represents a first internal standard voltage value, and $Z_{O1}$ (default = 800 Ω) represents a first external standard impedance value,

(ii) a second external standard impedance computing equation:

$$Z_{O2} = 200 = c \times V_{O2}/V_{R2} + os$$

wherein c and os represent variation constants based on impedance variation factors, $V_{O2}$ represents a second external standard voltage value, $V_{R2}$ represents a second internal standard voltage value, and $Z_{O2}$ (default = 200 Ω) represents a second external standard impedance value,

(iii) an inclination computing equation:

$$p = \{(Z_{O3} + Z_{O1} + Z_{O3}) - Z_{O2}\}/(V_{R3} - V_{R2})$$

$$= \{(800 + 800 + 800) - 200\}/(V_{R3} - V_{R2})$$

wherein $Z_{O1}$ (default = 800 Ω) represents a first external standard impedance value, $Z_{O3}$ (default = 800 Ω) represents a third external standard impedance value, $Z_{O2}$ (default = 200 Ω) represents a second external standard impedance value, $V_{R3}$ represents a third internal standard voltage value, $V_{R2}$ represents a second internal standard voltage value, and p represents an inclination constant of an internal external standard impedance relationship,

(iv) a total impedance computing equation:

$$Z_{TOTAL} = p \times V_{R4} + q$$

wherein p represents an inclination constant of an internal external standard impedance relationship, $V_{R4}$ represents a fourth internal standard voltage value, q (= second internal standard voltage value $V_{R2}$) represents a section constant of the internal external standard impedance relationship, and $Z_{TOTAL}$ represents a total impedance value in measuring a body,

(v) a first provisional internal impedance computing equation:

$$Z_{B-TEMP} = c \times V_{HUM}/V_{R4} + os$$

wherein c and os represent variation constants based on impedance variation factors, $V_{HUM}$ represents a body voltage value, $V_{R4}$ represents a fourth internal standard voltage value, and $Z_{B-TEMP}$ represents a provisional internal impedance value in measuring a body,

(vi) a body contact impedance computing equation:

$$Z_C = (Z_{TOTAL} - Z_{B-TEMP})/2$$

wherein $Z_{TOTAL}$ represents a total impedance value, $Z_{B-TEMP}$ represents a provisional internal impedance value in measuring a body, and $Z_C$ represents a contact impedance value in measuring the body,

(vii) a second provisional internal impedance computing equation:

$$Z_{O1-TEMP} = c \times V_{O3}/V_{R3} + os$$

wherein c and os represent variation constants based on impedance variation factors, $V_{R3}$ represents a third internal standard voltage value, $V_{O3}$ represents a third external standard voltage value, and $Z_{O1-TEMP}$ represents a provisional internal impedance value in measuring a first external standard impedance and a third external standard impedance,

(viii) an external standard contact impedance computing equation:

$$Z_{CO} = \{(Z_{O3} + Z_{O1} + Z_{O3}) - Z_{O1-TEMP}\}/2$$

$$= \{(800 + 800 + 800) - Z_{O1-TEMP}\}/2$$

wherein $Z_{O1}$ (default = 800 Ω) represents a first external standard impedance value, $Z_{O3}$ (default = 800 Ω) represents a third external standard impedance, $Z_{O1-TEMP}$ represents a provisional internal impedance value in measuring a first external standard impedance and a third external standard impedance, and $Z_{CO}$ represents a contact impedance value in measuring an external standard,

(ix) an external standard impedance computing equation:

$$Z_{O1} = 800 = (1 + k \times Z_{CO}) \times Z_{O1\text{-}TEMP}$$

wherein k represents a correction constant, $Z_{CO}$ represents a contact impedance value in measuring an external standard, $Z_{O1\text{-}TEMP}$ represents a provisional internal impedance value in measuring a first external standard impedance and a third external standard impedance, and $Z_{O1}$ represents a first external standard impedance value,

(x) an internal impedance computing equation:

$$Z_B = (1 + k \times \dot{Z}_C) \times Z_{B\text{-}TEMP}$$

wherein k represents a correction constant, $Z_C$ represents a contact impedance value in measuring a body, $Z_{B\text{-}TEMP}$ represents a provisional internal impedance value in measuring the body, and $Z_B$ represents an internal impedance value in measuring the body,

(xi) a biological equivalent model associated parameter computing equation:

$$Z_B \ (= R_B + j \times X_B) = Re(Ri + 1/2 \times \pi \times f \times j \times Cm)$$
$$/Re + (Ri + 1/2 \times \pi \times f \times j \times Cm)$$

wherein Re represents an extracellular fluid resistance, Ri represents an intracellular fluid resistance, Cm represents distributed membrane capacitance, f represents a frequency, j represents an imaginary number, $\pi$ represents a pi, and $Z_B$ represents an internal impedance ($R_B$ represents a resistance component, and $X_B$ represents a reactance component), and

(xii) skin condition computing equations:

$$Fw = a_{w1} \times R_{C50}^{-1} + a_{w2} \times Re^{-1} + a_{w3} \times Ri/Re + a_{w4}$$

$$Ft = a_{t1} \times Re^{-1} + a_{t2} \times R_\infty^{-1} + a_{t3} \times Ri^{-1} + a_{t4} \times \phi + a_{t5}$$

$$Fs = a_{s1} \times \phi + a_{s2} \times X_{B50}/R_{B50} + a_{s3}$$

$$Ff = a_{f1} \times R_{B6.25}/X_{B6.25} + a_{f2} \times R_{C50}/X_{C50} + a_{f3}$$

wherein Fw represents moisture (or dryness), Ft represents resilience (or swelling), Fs represents texture, Ff represents oiliness (or gloss), $R_{C50}$ represents the resistance component of a contact impedance with an alternating current of 50 kHz, $X_{C50}$ represents the reactance component of the contact impedance with the alternating current of 50 kHz, $R_{B50}$ represents the resistance component of an internal impedance with an alternating current of 50 kHz, $X_{B50}$ represents the reactance component of the internal impedance with the alternating current of 50 kHz, $R_{B6.25}$ represents the resistance component of an internal impedance with an alternating current of 6.25 kHz, $X_{B6.25}$ represents the reactance component of the internal impedance with the alternating current of 6.25 kHz, Re represents an extracellular fluid resistance, Ri represents an intracellular fluid resistance, $R_\infty$ represents a parallel combined resistance of the extracellular fluid resistance and the intracellular fluid resistance, $\phi$ represents an angle formed by the real part axis and a straight line which connects the real part axis with an internal impedance measured with an alternating current of 50 kHz when an arc corresponding to internal impedances measured by the above impedance measuring means using alternating currents of multiple frequencies is drawn on the real part axis and the imaginary part axis based on the arc law representing the relationship of impedances dependent on frequencies, and $a_{w1}$ to $a_{w4}$, $a_{t1}$ to $a_{t5}$, $a_{s1}$ to $a_{s3}$ and $a_{f1}$ to $a_{f3}$ represent coefficients (constants), in the computing equation storing section 17 in advance. Further, the EEPROM 2 also stores a second internal standard voltage value $V_{R2}$ which has been measured in the measuring section 1 and variation constants c and os based on impedance variation factors, an inclination constant p of an internal external standard impedance relationship and a correction constant k which have been computed in a constant computing section 19 in the constant storing section 18.

The above first external standard impedance computing equation, second external standard impedance computing equation, first provisional internal impedance computing equation and second provisional internal impedance computing equation are derived from the circuit model in Fig. 6 taking into consideration variations based on impedance variation factors which occur in the measuring section 1. In Fig. 6, $Z_R$ represents an internal standard impedance, $Z_C$ represents a contact impedance in measuring a body, $Z_B$ represents an internal impedance in measuring the body, $Z_{TOTAL}$ represents a total impedance in measuring the body, $Z_S$ represents an impedance by stray capacitance or the like, $Z_P$ represents a parasitic impedance, $V_O$ represents a voltage occurring over the internal standard impedance and the body, $I_C$ represents a constant current, and AMP represents a circuit related to voltage detection. The variation constant c based on impedance variation factors is a scale factor variation constant, and the variation constant os based on impedance variation factors is an offset voltage variation factor. Further, the biological equivalent model associated parameter computing equation is derived from the bioelectrical equivalent model in Fig. 2 assumed from a fact that the frequency domain in passing a current through a living body is attributable to a biological structure of extracellular electrolyte/cell membrane/intracellular electrolyte. Further, the skin condition computing equations are derived by classifying skin conditions into sensory characteristics (moisture (or dryness), resilience (or swelling), texture, oiliness (or gloss)) and determining the relationships in between these sensory characteristics and resistance and reactance components in contact and internal impedances and parameters (Re, Ri, Cm, $R_\infty$, $\phi$) associated with an equivalent model constituting a living tissue for a number of subjects.

Of these sections constituting the skin condition estimating apparatus, the microcontroller 3 comprises the constant computing section 19 which computes the variation constants, inclination constant and correction constant, a contact impedance computing section 20 which computes the total impedance value in measuring the body, the provisional internal impedance value in measuring the body, the contact impedance value in measuring the body, the provisional internal impedance value in measuring the first external standard impedance and the third external impedance and the contact impedance value in measuring the external standard impedance, an internal impedance computing section 21 which computes the internal impedance value in measuring the body, a biological equivalent model associated parameter computing section 22 which computes the parameters associated with the equivalent model constituting the living tissue, and a skin condition computing section 23 which computes a skin condition and performs various computations, controls and internal storages. Specific operations in the constant computing section 19, the contact impedance computing section 20, the internal impedance computing section 21, the biological equivalent model associated parameter computing section 22 and the skin condition computing section 23 will be described in the following description of the operations of the skin condition estimating apparatus according to the present invention.

Of these sections constituting the skin condition estimating apparatus, the display section 4 displays results computed by the microcontroller 3 and other data. The input section 6 turns on power and switches to a constant acquiring mode. The power supply section 7 supplies electric power to each section of the electrical system based on a signal from the input section 6.

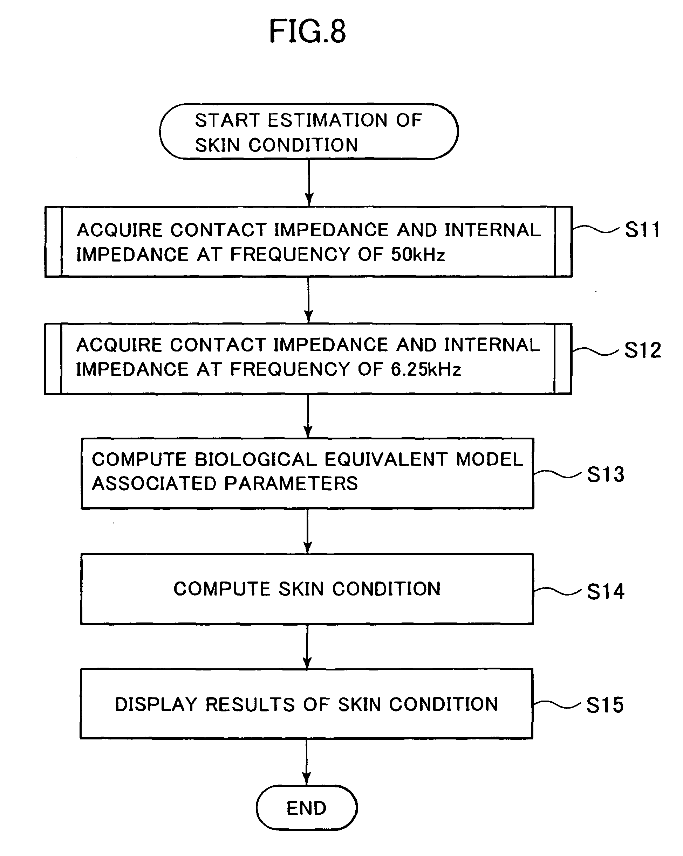

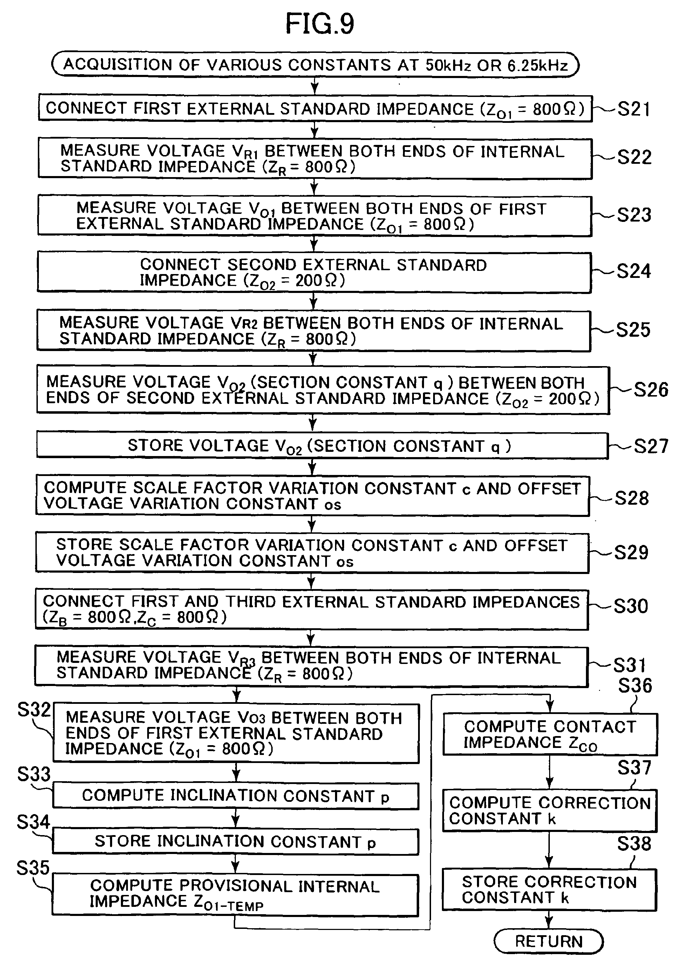

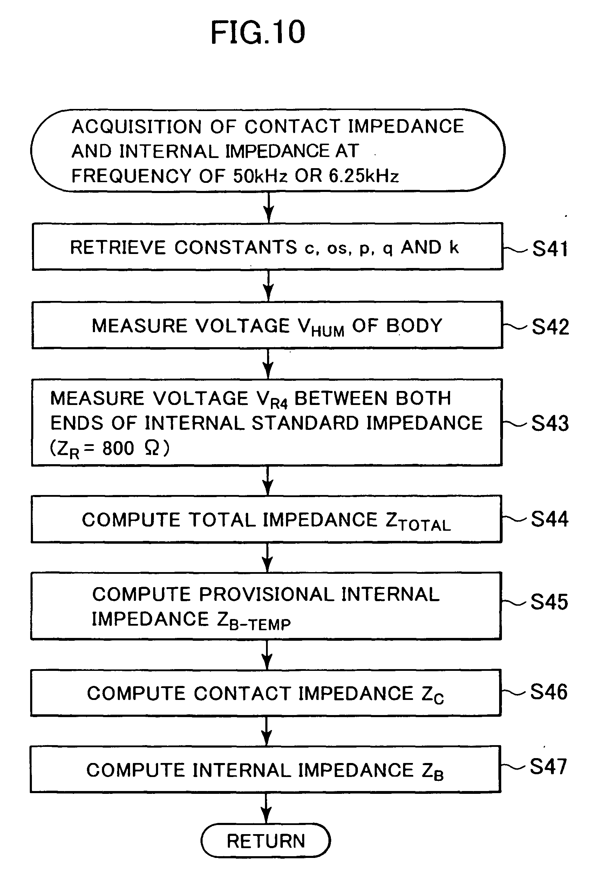

Next, specific operations of the skin condition estimating apparatus according to the present invention will be described by use of a main flowchart in the constant acquiring mode shown in Fig. 7, a main flowchart in a measuring mode shown in Fig. 8, a subroutine flowchart in the constant acquiring mode shown in Fig. 9 and a subroutine flowchart in the measuring mode shown in Fig. 10.

The skin condition estimating apparatus acquires various constants prior to measurements of contact impedance and internal impedance. In accordance with the flowchart shown in Fig. 7, the apparatus acquires various constants in making measurements with an alternating current of 50 kHz (STEP S1) first and then acquires various constants in making measurements with an alternating current of 6.25 kHz (STEP S2).

In these steps, the same operations are performed in accordance with the flowchart shown in Fig. 9 at different frequencies.

First, when the skin condition estimating apparatus is switched to the constant acquiring mode by the input section 6 and only the first external standard impedance ($Z_{O1}$ = 800 $\Omega$) is connected to the electrodes A and B (STEP S21), a voltage value between both ends (i.e., between m and n in Fig. 5) of the internal standard impedance ($Z_R$ = 800 $\Omega$) is measured as the first internal standard voltage value $V_{R1}$ (STEP S22) and a voltage value between both ends (i.e., between b and c in Fig. 5) of the first external standard impedance ($Z_{O1}$ = 800 $\Omega$) is measured as the first external standard voltage value $V_{O1}$ (STEP S23) in the measuring section 1.

Then, when only the second external standard impedance ($Z_{O2}$ = 200 $\Omega$) is connected to the electrodes A and B (STEP S24), a voltage value between both ends (i.e., between m and n in Fig. 5) of the internal standard impedance ($Z_R$ = 800 $\Omega$) is measured as the second internal standard voltage value $V_{R2}$ (STEP S25) and a voltage value between both ends (i.e., between f and g in Fig. 5) of the second external standard impedance ($Z_{O2}$ = 200 $\Omega$) is measured as the second external standard voltage value $V_{O2}$ (STEP S26) in the measuring section 1. The second external standard voltage value $V_{O2}$ is stored in the constant storing section 18 (STEP S27).

Then, in the constant computing section 19, the first external standard voltage value $V_{O1}$ and the first internal standard voltage value $V_{R1}$ which have been measured in the measuring section 1 are substituted into the first external standard impedance computing equation stored in advance in the computing equation storing section 17, the second external standard voltage value $V_{O2}$ and the second internal standard voltage value $V_{R2}$ which have been measured in the measuring section 1 are substituted into the second external standard impedance computing equation stored in the computing equation storing section 17, and the two computing equations are solved as simultaneous equations so as to compute the variation constants c and os based on impedance variation factors (STEP S28). The variation constants c and os based on impedance variation factors are stored in the constant storing section 18 (STEP S29).

Then, when the first external standard impedance ($Z_{O1}$ = 800 $\Omega$) and the third external standard impedance ($Z_{O3}$ = 800 $\Omega$) are connected to the electrodes A and B (STEP S30), a voltage value between both ends (i.e., between m and n in Fig. 5) of the internal standard impedance ($Z_R$ = 800 $\Omega$) is measured as the third internal standard voltage value $V_{R3}$ (STEP S31) and a voltage value between both ends (i.e., between j and k in Fig. 5) of the first external standard impedance ($Z_{O1}$ = 800 $\Omega$) is measured as the third external standard voltage value $V_{O3}$ (STEP S32) in the measuring section 1.

Then, in the constant computing section 19, the third internal standard voltage value $V_{R3}$ and the second internal standard voltage value $V_{R2}$ which have been measured in the measuring section 1 are substituted into the inclination computing equation stored in the computing equation storing section 17 so as to compute the inclination constant p (STEP S33). The inclination constant p is stored in the constant storing section 18 (STEP S34).

Then, in the contact impedance computing section 20, the third external standard voltage value $V_{O3}$ and the third internal standard voltage value $V_{R3}$ which have been measured in the measuring section 1 and the variation constants c and os based on impedance variation factors which are stored in the constant storing section 18 are substituted into the second provisional internal impedance computing equation stored in the computing equation storing section 17 so as to compute the provisional internal impedance value $Z_{O1-TEMP}$ in measuring the first external standard impedance and the third external standard impedance (STEP S35).

Then, in the contact impedance computing section 20, the provisional internal impedance value $Z_{O1-TEMP}$ in measuring the first external standard impedance and the third external standard impedance is substituted into the external standard contact impedance computing equation stored in the computing equation storing section 17 so as to compute the contact impedance value $Z_{CO}$ in measuring the external standard in measuring the first external standard impedance and the third external standard impedance (STEP S36).

Then, in the constant computing section 19, the contact impedance value $Z_{CO}$ and the provisional internal impedance value $Z_{O1-TEMP}$ which have been computed in the contact impedance computing section 20 are substituted into the external standard impedance computing equation stored in the computing equation storing section 17 so as to compute the correction constant k (STEP S37) which is then stored in the constant storing section 18 (STEP S38), thereby ending the constant acquiring mode.

After acquiring various constants required to determine a contact impedance value and an internal impedance value, the skin condition estimating apparatus executes processes to determine a skin condition in accordance with the flowcharts shown in Figs. 8 and 10, unless switched to the constant acquiring mode.

As shown in the flowchart of Fig. 8, in the impedance measuring means, a contact impedance value $Z_{C50}$ ($R_{C50}$, $X_{C50}$) and an internal impedance value $Z_{B50}$ ($R_{B50}$, $X_{B50}$) in making measurements with an alternating current of 50 kHz are acquired (STEP S11), and a contact impedance value $Z_{C6.25}$ ($R_{C6.25}$. $X_{C6.25}$) and an internal impedance value $Z_{B6.25}$ ($R_{B6.25}$, $X_{B6.25}$) in making measurements with an alternating current of 6.25 kHz are acquired (STEP S12). Details of these acquiring processes will be described later by use of Fig. 10.

Then, in the biological equivalent model associated parameter computing section 22, the internal impedance value $Z_{B50}$ ($R_{B50}$, $X_{B50}$) or $Z_{B6.25}$ ($R_{B6.25}$, $X_{B6.25}$) acquired by the impedance measuring means is substituted into the internal impedance $Z_B$ (= $R_B$ + j $\times$ $X_B$) of the biological equivalent model associated parameter computing equation stored in the computing equation storing section 17 and a frequency of 50 kHz or 6.25 kHz is substituted into the frequency f of the above equation so as to formulate four equations, and the four equations are solved as simultaneous equations so as to compute the values of the extracellular fluid resistance Re, intracellular fluid resistance Ri and distributed membrane capacitance Cm. Further, the value of an angle $\phi$ ($\phi_1$ or $\phi_2$) formed by the real part axis and a straight line which connects the real part axis with the internal impedance $Z_{B50}$ measured when a vector locus corresponding to internal impedances measured by the impedance measuring means using alternating currents of multiple frequencies (50 kHz and 6.25 kHz) as shown in Fig. 3 is drawn on the real part axis and the imaginary part axis based on the arc law (cole-cole's arc law) representing a vector locus of impedances dependent on frequencies, and the value of the parallel combined resistance $R_\infty$ (= Re//Ri) of the extracellular fluid resistance and the intracellular fluid resistance are calculated (STEP S13).

Then, in the skin condition computing section 23, the values of the resistance components $R_{C50}$ and $R_{C6.25}$ and reactance components $X_{C50}$ and $X_{C6.25}$ of the contact impedances and the resistance components $R_{B50}$ and $R_{B6.25}$ and reactance components $X_{B50}$ and $X_{B6.25}$ of the internal impedances which have been acquired by the impedance measuring means and the values of the extracellular fluid resistance Re, intracellular fluid resistance Ri, distributed membrane capacitance Cm, angle $\phi$ ($\phi_1$ or $\phi_2$) and parallel combined resistance $R_\infty$ (= Re//Ri) which have been computed in the biological equivalent model associated parameter computing section 22 are substituted into the skin condition computing equations stored in the computing equation storing section 17 so as to compute the values of the moisture (or dryness) Fw, resilience (or swelling) Ft, texture Fs and oiliness (or gloss) Ff (STEP S14). These values are displayed on the display section 4 (STEP S15), thereby ending a series of operations.

In the above STEPS S11 and S12 in the flowchart of Fig. 8, the same operations are performed in accordance with the flowchart shown in Fig. 10 at different frequencies.

First, immediately after power is turn on or after the constant acquiring mode, the skin condition estimating apparatus enters the measuring mode and retrieves the variation constants c and os, the inclination constant p, the section constant q and the correction constant k which are stored in the constant storing section 18 in the contact impedance computing section 20 (STEP S41).

Then, when the grip 52 is applied to the skin so that the skin makes contact with the electrodes, the voltage value of the skin layer is measured as the body voltage value $V_{HUM}$ (STEP S42) and a voltage value between both ends (i.e., between m and n in Fig. 1) of the internal standard impedance ($Z_R$ = 800 $\Omega$) is measured as the fourth internal standard voltage value $V_{R4}$ (STEP S43) in the measuring section 1.

Then, in the contact impedance computing section 20, the fourth internal standard voltage value $V_{R4}$ measured in the measuring section 1 and the inclination constant p and the section constant q (= second internal standard voltage value $V_{R2}$) which have been retrieved from the constant storing section 18 are substituted into the total impedance computing equation stored in advance in the computing equation storing section so as to compute the total impedance value $Z_{TOTAL}$ in measuring the body (STEP S44).

Then, in the contact impedance computing section 20, the body voltage value $V_{HUM}$ and the fourth internal standard voltage value $V_{R4}$ which have been measured in the measuring section 1 and the variation constants c and os based on impedance variation factors which have been retrieved from the constant storing section 18 are substituted into the first provisional internal impedance computing equation stored in advance in the computing equation storing section so as to compute the provisional internal impedance value $Z_{B-TEMP}$ in measuring the body (STEP S45).

Then, in the contact impedance computing section 20, the above computed total impedance value $Z_{TOTAL}$ and provisional internal impedance value $Z_{B-TEMP}$ in measuring the body are substituted into the body contact impedance computing equation stored in advance in the computing equation storing section so as to compute the contact impedance value $Z_C$ in measuring the body.

Then, in the internal impedance computing section 21, the contact impedance value $Z_C$ in measuring the body and the provisional internal impedance value $Z_{B-TEMP}$ in measuring the body which have been computed in the contact impedance computing section 20 and the correction constant k retrieved from the constant storing section 18 are substituted into the internal impedance computing equation stored in advance in the computing equation storing section so as to compute the internal impedance value $Z_B$ in measuring the body (STEP S47) .

**Claims**

1.  A skin condition estimating apparatus comprising:

    impedance measuring means (101),
    biological equivalent model associated parameter estimating means (102), and
    skin condition estimating means (103),
    wherein
    the impedance measuring means (101) has electrodes (11, 12) to make contact with a body and is adapted to pass alternating currents of multiple frequencies through the body when the body is in contact with the electrodes (11, 12) to measure a contact impedance and an internal impedance which occur during the energization,
    the biological equivalent model associated parameter estimating means (102) is adapted to estimate parameters associated with an equivalent model constituting a living tissue based on the frequencies of the currents passed by the impedance measuring means (101) and the measured internal impedance,
    the skin condition estimating means (103) is adapted to estimate a skin condition based on at least one of the contact impedance measured by the impedance measuring means (101), the internal impedance measured by the impedance measuring means (101) and the parameters estimated by the biological equivalent model associated parameter estimating means (102),

**characterized in that**

the parameters associated with the equivalent model constituting the living tissue are an extracellular fluid resistance, an intracellular fluid resistance, distributed membrane capacitance, a parallel combined resistance of the extracellular fluid resistance and the intracellular fluid resistance, and an angle formed by the real part axis and a straight line which connects the real part axis with an internal impedance measured when a vector locus corresponding to the internal impedances measured by the impedance measuring means (101) using the alternating currents of multiple frequencies is drawn on the real part axis and the imaginary part axis based on the arc law representing a vector locus of impedances dependent on frequencies,

the alternating currents of multiple frequencies are an alternating current of 50 kHz and an alternating current of 6.25 kHz,

the skin condition estimating means (103) is adapted to estimate at least one characteristic selected from the group consisting of moisture or dryness, resilience or swelling, texture and oiliness or gloss as a skin condition, and

the skin condition estimating means (103) is adapted to estimate moisture or dryness, resilience or swelling, texture and oiliness or gloss by use of the following equations:

$$Fw = a_{w1} \times R_{C50}^{-1} + a_{w2} \times Re^{-1} + a_{w3} \times Ri/Re + a_{w4}$$

$$Ft = a_{t1} \times Re^{-1} + a_{t2} \times R_{\infty}^{-1} + a_{t3} \times Ri^{-1} + a_{t4} \times \Phi + a_{t5}$$

$$Fs = a_{s1} \times \Phi + a_{s2} \times X_{B50}/R_{B50} + a_{s3}$$

$$Ff = a_{f1} \times R_{B6.25}/X_{B6.25} + a_{f2} \times R_{C50}/X_{C50} + a_{f3}$$

wherein Fw represents moisture or dryness, Ft represents resilience or swelling, Fs represents texture, Ff represents oiliness or gloss, $R_{C50}$ represents the resistance component of a contract impedance with an alternating current of 50 kHz, $X_{C50}$ represents the reactance component of the contact impedance with the alternating current of 50 kHz, $R_{B50}$ represents the resistance component of an internal impedance with an alternating current of 50 kHz, $X_{B50}$ represents the reactance component of the internal impedance with the alternating current of 50 kHz, $R_{B6.25}$ represents the resistance component of an internal impedance with an alternating current of 6.25 kHz, $X_{B6.25}$ represents the reactance component of the internal impedance with the alternating current of 6.25 kHz, Re represents an extracellular fluid resistance, Ri represents an intracellular fluid resistance, $R_{\infty}$ represents a parallel combined resistance of the extracellular fluid resistance and the intracellular fluid resistance, $\phi$ represents an angle formed by the real part axis and a straight line which connects the real part axis with an internal impedance measured with an alternating current of 50 kHz when an arc corresponding to the internal impedances measured by the impedance measuring means (101) using the alternating currents of multiple frequencies is drawn on the real part axis and the imaginary part axis based on the arc law representing the relationship of impedances dependent on frequencies, and $a_{w1}$ to $a_{w4}$, $a_{t1}$ to $a_{t5}$, $a_{s1}$ to $a_{s3}$ and $a_{f1}$ to $a_{t3}$ represent coefficients.

2. The apparatus of claim 1, wherein the biological equivalent model associated parameter estimating means (102) is adapted to estimate the extracellular fluid resistance, the intracellular fluid resistance and the distributed membrane capacitance by use of the following equation:

$$Z_B = R_B + j \times X_B = Re(Ri + 1/2 \times \pi \times f \times j \times Cm)$$
$$/Re + (Ri + 1/2 \times \pi \times f \times j \times Cm)$$

wherein Re represents an extracellular fluid resistance, Ri represents an intracellular fluid resistance, Cm represents distributed membrane capacitance, f represents a frequency, j represents an imaginary number, $\pi$ represents a pi, $Z_B$ represents an internal impedance, $R_B$ represents a resistance component and $X_B$ represents a reactance component.

**Patentansprüche**

1. Hautzustand-Bestimmungsvorrichtung, umfassend:

Impedanzmessmittel (101),
Mittel (102) zur Bestimmung von mit einem biologischen Äquivalentmodell verbundenen Parametern, und
Hautzustand-Bestimmungsmittel (103),
worin
das Impedanzmessmittel (101) Elektroden (11, 12) zum Herstellen von Kontakt mit einem Körper aufweist und geeignet ist, Wechselströme mit mehreren Frequenzen durch den Körper zu leiten, wenn der Körper in Kontakt mit den Elektroden (11, 12) ist, um eine Kontaktimpedanz und eine Innenimpedanz zu messen, die während der Stromzufuhr auftreten,
das Mittel (102) zur Bestimmung von mit einem biologischen Äquivalentmodell verbundenen Parametern dazu geeignet ist, Parameter zu bestimmen, die mit einem Äquivalentmodell verbunden sind, welches ein lebendes Gewebe darstellt, basierend auf den Frequenzen der Ströme, die durch das Impedanzmessmittel (101) durch den Körper geleitet werden, und auf der gemessenen Innenimpedanz,
das Hautzustand-Bestimmungsmittel (103) dazu geeignet ist, den Hautzustand basierend auf wenigstens einem von der Kontaktimpedanz, die durch das Impedanzmessmittel (101) gemessen wurde, der Innenimpedanz, die durch das Impedanzmessmittel (101) gemessen wurde, und den Parametern, die durch das Mittel (102) zur Bestimmung von mit einem biologischen Äquivalentmodell verbundenen Parametern bestimmt wurden, zu bestimmen,
**dadurch gekennzeichnet, dass**
die Parameter, die mit dem Äquivalentmodell verbunden sind, welches das lebende Gewebe darstellt, ein extrazellulärer Fluidwiderstand, ein intrazellulärer Fluidwiderstand, eine Membrankapazität, ein parallel kombinierter Widerstand aus dem extrazellulären Fluidwiderstand und dem intrazellulären Fluidwiderstand, und ein Winkel sind, der aus der Realteilachse und einer geraden Linie gebildet wird, welche die Realteilachse mit einer Innenimpedanz verbindet, wenn ein Vektorort entsprechend der durch das Impedanzmessmittel (101) unter Verwendung der Wechselströme mit mehreren Frequenzen gemessenen Innenimpedanz auf der Realteilachse und der Imaginärteilachse aufgetragen wird, basierend auf dem Bogengesetz gemessen, welches einen Vektorort von Impedanzen in Abhängigkeit von den Frequenzen darstellt,
die Wechselströme mit mehreren Frequenzen ein Wechselstrom von 50 kHz und ein Wechselstrom von 6,25 kHz sind,
das Hautzustand-Bestimmungsmittel (103) dazu geeignet ist, wenigstens eine Eigenschaft aus der Gruppe von Feuchtigkeit oder Trockenheit, Elastizität oder Schwellung, Textur und Fettigkeit oder Glanz als einen Hautzustand zu bestimmen, und
das Hautzustand-Bestimmungsmittel (103) dazu geeignet ist, Feuchtigkeit oder Trockenheit, Elastizität oder Schwellung, Textur und Fettigkeit oder Glanz unter Verwendung der folgenden Gleichungen zu berechnen:

$$Fw = a_{w1} \times R_{C50}^{-1} + a_{w2} \times Re^{-1} + a_{w3} \times Ri/Re + a_{w4}$$

$$Ft = a_{t1} \times Re^{-1} + a_{t2} \times R_{\infty}^{-1} + a_{t3} \times Ri^{-1} + a_{t4} \times \Phi + a_{t5}$$

$$Fs = a_{s1} \times \Phi + a_{s2} \times X_{B50}/R_{B50} + a_{s3}$$

$$Ff = a_{f1} \times R_{B6.25}/X_{B6.25} + a_{f2} \times R_{C50}/X_{C50} + a_{f3}$$

worin Fw Feuchtigkeit oder Trockenheit darstellt, Ft Elastizität oder Schwellung darstellt, Fs Textur darstellt, Ff Fettigkeit oder Glanz darstellt, $R_{C50}$ die Widerstandskomponente einer Kontaktimpedanz mit einem Wechselstrom von 50 kHz darstellt, $X_{C50}$ die Reaktänzkömponente der Köntaktimpedanz mit einem Wechselstrom von 50 kHz darstellt, $R_{B50}$ die Widerstandskomponente einer Innenimpedanz mit einem Wechselstrom von 50 kHz darstellt, $X_{B50}$ die Reaktanzkomponente der Innenimpedanz mit einem Wechselstrom von 50 kHz darstellt, $R_{B6.25}$ die Widerstandskomponente einer Innenimpedanz mit einem Wechselstrom von 6,25 kHz darstellt, $X_{B6.25}$ die Reaktanzkomponente der Innenimpedanz mit einem Wechselstrom von 6,25 kHz darstellt, Re den extrazellulären Fluidwiderstand darstellt, Ri den intrazellulären Fluidwiderstand darstellt, $R_\infty$ den parallel kombinierten Widerstand aus dem extrazellulären Fluidwiderstand und dem intrazellulären Fluidwiderstand darstellt, $\phi$ einen Winkel darstellt, der aus der Realteilachse und einer geraden Linie gebildet wird, welche die Realteilachse mit einer Innenimpedanz verbindet, gemessen mit einer Frequenz von 50 kHz, wenn ein Bogen entsprechend der durch das Impedanzmessmittel (101) unter Verwendung der Wechselströme mit mehreren Frequenzen gemessenen Innenimpedanz auf der Realteilachse und der Imaginärteilachse aufgetragen wird, basierend auf dem Bogengesetz, welches die Beziehung von Impedanzen in Abhängigkeit von den Frequenzen darstellt, und $a_{w1}$ bis $a_{w4}$, $a_{t1}$ bis $at_5$, $a_{s1}$ bis $a_{s3}$ und $a_{f1}$ bis $a_{f3}$ Koeffizienten darstellen.

2. Vorrichtung nach Anspruch 1, worin das Mittel (102) zur Bestimmung von mit einem biologischen Äquivalentmodell verbundenen Parametern dazu geeignet ist, den extrazellulären Fluidwiderstand, den intrazellulären Fluidwiderstand und die Membrankapazität unter Verwendung der folgenden Gleichung zu bestimmen:

$$Z_B = R_B + j \times X_B = Re(Ri + 1/2 \times \pi \times f \times j \times Cm)$$
$$/Re + (Ri + 1/2 \times \pi \times f \times j \times Cm)$$

worin Re den extrazellulären Fluidwiderstand darstellt, Ri den intrazellulären Fluidwiderstand darstellt, Cm die Membrankapazität darstellt, f eine Frequenz darstellt, j eine imaginäre Zahl darstellt, $\pi$ die Zahl Pi darstellt, $Z_B$ eine Innenimpedanz darstellt, $R_B$ eine Widerstandskomponente darstellt und $X_B$ eine Reaktanzkomponente darstellt.

**Revendications**

1. Dispositif d'estimation de l'état de la peau comprenant :

un moyen de mesure d'impédance (101),
un moyen d'estimation de paramètres associés à un modèle équivalent biologique (102), et
un moyen d'estimation de l'état de la peau (103),
où
le moyen de mesure d'impédance (101) comporte des électrodes (11, 12) pour réaliser un contact avec un corps et est conçu pour faire passer des courants alternatifs de fréquences multiples à travers le corps lorsque le corps est en contact avec les électrodes (11, 12) pour mesurer une impédance de contact et une impédance interne qui apparaissent durant l'excitation,
le moyen d'estimation de paramètres associés à un modèle équivalent biologique (102) est conçu pour estimer des paramètres associés à un modèle équivalent constituant un tissu vivant sur la base des fréquences des courants transmis par le moyen de mesure d'impédance (101) et l'impédance interne mesurée,
le moyen d'estimation de l'état de la peau (103) est conçu pour estimer l'état de la peau sur la base d'au moins l'un de l'impédance de contact mesurée par le moyen de mesure d'impédance (101), de l'impédance interne mesurée par le moyen de mesure d'impédance (101) et des paramètres estimés par le moyen d'estimation de paramètres associés à un modèle équivalent biologique (102),
**caractérisé en ce que**
les paramètres associés au modèle équivalent constituant le tissu vivant sont une résistance de fluide extracellulaire, une résistance de fluide intracellulaire, une capacité de membrane répartie, une résistance combinée parallèle constituée de la résistance de fluide extracellulaire et de la résistance de fluide intracellulaire, et un angle formé par l'axe de partie réelle et une ligne droite qui relie l'axe de partie réelle avec une impédance interne mesurée lorsqu'un lieu géométrique de vecteur correspondant aux impédances internes mesurées par le moyen de mesure d'impédance (101) en utilisant les courants alternatifs de fréquences multiples est tracé sur l'axe de partie réelle et l'axe de partie imaginaire sur la base de la loi d'arc représentant un lieu géométrique de vecteur des impédances en fonction des fréquences,

les courants alternatifs de fréquences multiples sont un courant alternatif de 50 kHz et un courant alternatif de 6,25 kHz,

le moyen d'estimation de l'état de la peau (103) est conçu pour estimer au moins une caractéristique sélectionnée à partir du groupe constitué de la moiteur ou de la sécheresse, de l'élasticité ou du gonflement, de la texture et de l'aspect huileux ou de l'éclat en tant qu'état de la peau, et

le moyen d'estimation de l'état de la peau (103) est conçu pour estimer la moiteur ou la sécheresse, l'élasticité ou le gonflement, la texture et l'aspect huileux ou l'éclat par le biais de l'utilisation des équations suivantes :

$$Fw = a_{w1} \times R_{C50}^{-1} + a_{w2} \times Re^{-1} + a_{w3} \times Ri/Re + a_{w4}$$

$$Ft = a_{t1} \times Re^{-1} + a_{t2} \times R_\infty^{-1} + a_{t3} \times Ri^{-1} + a_{t4} \times \Phi + a_{t5}$$

$$Fs = a_{s1} \times \Phi + a_{s2} \times X_{B50}/R_{B50} + a_{s3}$$

$$Ff = a_{f1} \times R_{B6,25}/X_{B6,25} + a_{f2} \times R_{C50}/X_{C50} + a_{f3}$$

où Fw représente la moiteur ou la sécheresse, Ft représente l'élasticité ou le gonflement, Fs représente la texture, Ff représente l'aspect huileux ou l'éclat, $R_{C50}$ représente la composante de résistance d'une impédance de contact avec un courant alternatif de 50 kHz, $X_{C50}$ représente la composante de réactance de l'impédance de contact avec le courant alternatif de 50 kHz, $R_{B50}$ représente la composante de résistance d'une impédance interne avec un courant alternatif de 50 kHz, $X_{B50}$ représente la composante de réactance de l'impédance interne avec le courant alternatif de 50 kHz, $R_{B6,25}$ représente la composante de résistance d'une impédance interne avec un courant alternatif de 6,25 kHz, $X_{B6,25}$ représente la composante de réactance de l'impédance interne avec le courant alternatif de 6,25 kHz, Re représente une résistance de fluide extracellulaire, Ri représente une résistance de fluide intracellulaire, $R_\infty$ représente une résistance combinée parallèle constituée de la résistance de fluide extracellulaire et de la résistance de fluide intracellulaire, $\Phi$ représente un angle formé par l'axe de partie réelle et une ligne droite qui relie l'axe de partie réelle avec une impédance interne mesurée avec un courant alternatif de 50 kHz lorsqu'un arc correspondant aux impédances internes mesurées par le moyen de mesure d'impédance (101) utilisant les courants alternatif des fréquences multiples est tracé sur l'axe de partie réelle et l'axe de partie imaginaire sur la base de la loi d'arc représentant la relation des impédances en fonction des fréquences et $a_{w1}$ à $a_{w4}$, $a_{t1}$ à $a_{t5}$, $a_{s1}$ à $a_{s3}$ et $a_{f1}$ à $a_{f3}$ représentent des coefficients.

**2.** Dispositif selon la revendication 1, dans lequel le moyen d'estimation de paramètres associés à un modèle équivalent biologique (102) est conçu pour estimer la résistance de fluide extracellulaire, la résistance de fluide intracellulaire et la capacité de membrane répartie par le biais de l'utilisation de l'équation suivante :

$$Z_B = R_B + j \times X_B = Re(Ri + 1/2 \times \pi \times f \times j \times Cm)$$
$$/Re + (Ri + 1/2 \times \pi \times f \times j \times Cm)$$

où Re représente une résistance de fluide extracellulaire, Ri représente une résistance de fluide intracellulaire, Cm représente une capacité de membrane répartie, f représente une fréquence, j représente un nombre imaginaire, $\pi$ représente pi, $Z_B$ représente une impédance interne, $R_B$ représente une composante de résistance et $X_B$ représente une composante de réactance.

# FIG.1

```
┌─────────────────────────────────────────────────┐
│  IMPEDANCE MEASURING MEANS              ←─101     │
│                                                   │
│  ⎡ CONTACT IMPEDANCE  ⎤                            │
│  ⎣ INTERNAL IMPEDANCE ⎦                            │
└─────────────────────────────────────────────────┘
```

102

```
┌─────────────────────────────────────────────────┐
│  BIOLOGICAL EQUIVALENT MODEL ASSOCIATED           │
│  PARAMETER ESTIMATING MEANS                       │
│                                                   │
│  ⎡ EXTRACELLULAR FLUID RESISTANCE          ⎤      │
│  │ INTRACELLULAR FLUID RESISTANCE          │      │
│  │ DISTRIBUTED MEMBRANE CAPACITANCE        │      │
│  │ PARALLEL COMBINED RESISTANCE OF         │      │
│  │ INTRACELLULAR AND EXTRACELLULAR         │      │
│  ⎣ FLUID RESISTANCE                        ⎦      │
└─────────────────────────────────────────────────┘
```

```
┌─────────────────────────────────────────────────┐
│  SKIN CONDITION                                   │
│  ESTIMATING MEANS                                 │
│                                                   │
│  103   ⎡ MOISTURE (DRYNESS)     ⎤                 │
│        │ RESILIENCE (SWELLING)  │                 │
│        │ TEXTURE                │                 │
│        ⎣ OILINESS (GLOSS)       ⎦                 │
└─────────────────────────────────────────────────┘
```

# FIG.2

# FIG.3

# FIG.4

51

4

SETTING · SELEC-TION · POWER

6c · 6b · 6a

53 · 52 · 11 11 · 12 · 12

# FIG.5

BODY $Z_C$

$Z_B$

EXTERNAL STANDARD
IMPEDANCES

a b $Z_{O1}$ c d
e f $Z_{O2}$ g h
i $Z_{O3}$ j $Z_{O1}$ k $Z_{O3}$ l

1: MEASURING
SECTION

INTERNAL STANDARD
IMPEDANCE

m $Z_R$ n ~10

ELECTRODE A

ELECTRODE A

12

11

V/I CONVERTER ~9

ELECTRODE B

11

ELECTRODE B

8

12

14

8

SWITCHER

AMPLIFIER

CONSTANT VOLTAGE
(SINUSOIDAL ALTERNATING
CURRENT) GENERATOR

13

16

A/D CONVERTER

FILTER ~15

3

MICROCONTROLLER

7

POWER SUPPLY
SECTION

19 — CONSTANT COMPUTING SECTION.
20 — CONTACT IMPEDANCE
COMPUTING SECTION.
21 — INTERNAL IMPEDANCE
COMPUTING SECTION.
22 — BIOLOGICAL EQUIVALENT MODEL
ASSOCIATED PARAMETER
23 — COMPUTING SECTION.
2 — SKIN CONDITION COMPUTING SECTION.

6

INPUT SECTION

4

DISPLAY SECTION

EEPROM

17

COMPUTING EQUATION STORING SECTION

18

CONSTANT
STORING
SECTION

VARIATION
CONSTANTS

INCLINATION
CONSTANT

SECTION
CONSTANT

CORRECTION
CONSTANT

FIRST EXTERNAL STANDARD
IMPEDANCE COMPUTING EQUATION.
SECOND EXTERNAL STANDARD IMPEDANCE
COMPUTING EQUATION.
INCLINATION COMPUTING EQUATION.
TOTAL IMPEDANCE COMPUTING EQUATION.
FIRST PROVISIONAL INTERNAL IMPEDANCE
COMPUTING EQUATION.
BODY CONTACT IMPEDANCE
COMPUTING EQUATION.
SECOND PROVISIONAL INTERNAL IMPEDANCE
COMPUTING EQUATION.
EXTERNAL STANDARD CONTACT IMPEDANCE
COMPUTING EQUATION.
EXTERNAL STANDARD IMPEDANCE COMPUTING
EQUATION.
INTERNAL IMPEDANCE COMPUTING EQUATION.
BIOLOGICAL EQUIVALENT MODEL ASSOCIATED
PARAMETER COMPUTING EQUATION.
SKIN CONDITION COMPUTING EQUATIONS.

# FIG.6

# FIG.7

START ACQUISITION OF
VARIOUS CONSTANTS

ACQUIRE VARIOUS CONSTANTS
AT FREQUENCY OF 50kHz — S1

ACQUIRE VARIOUS CONSTANTS
AT FREQUENCY OF 6.25kHz — S2

END

# FIG.8

```
        ┌─────────────────────────┐
        │   START ESTIMATION OF   │
        │     SKIN CONDITION      │
        └─────────────────────────┘
                    │
                    ▼
  ┌──────────────────────────────────────────┐
  │ ACQUIRE CONTACT IMPEDANCE AND INTERNAL    │ ~~ S11
  │ IMPEDANCE AT FREQUENCY OF 50kHz           │
  └──────────────────────────────────────────┘
                    │
                    ▼
  ┌──────────────────────────────────────────┐
  │ ACQUIRE CONTACT IMPEDANCE AND INTERNAL    │ ~~ S12
  │ IMPEDANCE AT FREQUENCY OF 6.25kHz         │
  └──────────────────────────────────────────┘
                    │
                    ▼
  ┌──────────────────────────────────────────┐
  │ COMPUTE BIOLOGICAL EQUIVALENT MODEL       │ ~~ S13
  │ ASSOCIATED PARAMETERS                     │
  └──────────────────────────────────────────┘
                    │
                    ▼
  ┌──────────────────────────────────────────┐
  │        COMPUTE SKIN CONDITION            │ ~~ S14
  └──────────────────────────────────────────┘
                    │
                    ▼
  ┌──────────────────────────────────────────┐
  │   DISPLAY RESULTS OF SKIN CONDITION      │ ~~ S15
  └──────────────────────────────────────────┘
                    │
                    ▼
              ┌───────────┐
              │    END    │
              └───────────┘
```

# FIG.9

( ACQUISITION OF VARIOUS CONSTANTS AT 50kHz OR 6.25kHz )

↓

| CONNECT FIRST EXTERNAL STANDARD IMPEDANCE ($Z_{O1}$ = 800Ω) |—S21

↓

| MEASURE VOLTAGE $V_{R1}$ BETWEEN BOTH ENDS OF INTERNAL STANDARD IMPEDANCE ($Z_R$ = 800Ω) |—S22

↓

| MEASURE VOLTAGE $V_{O1}$ BETWEEN BOTH ENDS OF FIRST EXTERNAL STANDARD IMPEDANCE ($Z_{O1}$ = 800Ω) |—S23

↓

| CONNECT SECOND EXTERNAL STANDARD IMPEDANCE ($Z_{O2}$ = 200Ω) |—S24

↓

| MEASURE VOLTAGE $V_{R2}$ BETWEEN BOTH ENDS OF INTERNAL STANDARD IMPEDANCE ($Z_R$ = 800Ω) |—S25

↓

| MEASURE VOLTAGE $V_{O2}$ (SECTION CONSTANT q) BETWEEN BOTH ENDS OF SECOND EXTERNAL STANDARD IMPEDANCE ($Z_{O2}$ = 200Ω) |—S26

↓

| STORE VOLTAGE $V_{O2}$ (SECTION CONSTANT q) |—S27

↓

| COMPUTE SCALE FACTOR VARIATION CONSTANT c AND OFFSET VOLTAGE VARIATION CONSTANT os |—S28

↓

| STORE SCALE FACTOR VARIATION CONSTANT c AND OFFSET VOLTAGE VARIATION CONSTANT os |—S29

↓

| CONNECT FIRST AND THIRD EXTERNAL STANDARD IMPEDANCES ($Z_B$ = 800Ω,$Z_C$ = 800Ω) |—S30

↓

| MEASURE VOLTAGE $V_{R3}$ BETWEEN BOTH ENDS OF INTERNAL STANDARD IMPEDANCE ($Z_R$ = 800Ω) |—S31

↓

S32 | MEASURE VOLTAGE $V_{O3}$ BETWEEN BOTH ENDS OF FIRST EXTERNAL STANDARD IMPEDANCE ($Z_{O1}$ = 800Ω) |

S33 | COMPUTE INCLINATION CONSTANT p |

S34 | STORE INCLINATION CONSTANT p |

S35 | COMPUTE PROVISIONAL INTERNAL IMPEDANCE $Z_{O1-TEMP}$ |

S36 | COMPUTE CONTACT IMPEDANCE $Z_{CO}$ |

S37 | COMPUTE CORRECTION CONSTANT k |

S38 | STORE CORRECTION CONSTANT k |

↓

( RETURN )

# FIG.10

ACQUISITION OF CONTACT IMPEDANCE
AND INTERNAL IMPEDANCE AT
FREQUENCY OF 50kHz OR 6.25kHz

RETRIEVE CONSTANTS c, os, p, q AND k — S41

MEASURE VOLTAGE $V_{HUM}$ OF BODY — S42

MEASURE VOLTAGE $V_{R4}$ BETWEEN BOTH
ENDS OF INTERNAL STANDARD IMPEDANCE — S43
($Z_R$ = 800 Ω)

COMPUTE TOTAL IMPEDANCE $Z_{TOTAL}$ — S44

COMPUTE PROVISIONAL INTERNAL
IMPEDANCE $Z_{B-TEMP}$ — S45

COMPUTE CONTACT IMPEDANCE $Z_C$ — S46

COMPUTE INTERNAL IMPEDANCE $Z_B$ — S47

RETURN

**EP 1 576 922 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003169784 A
- JP 2003169785 A
- XP 008048739